# EUROPEAN PATENT SPECIFICATION

(11) **EP 4 110 189 B1**
(45) Date of publication and mention of the grant of the patent: **04.12.2024**
(21) Application number: 21708164.5
(22) Date of filing: 20.02.2021
(51) Int. Cl.: A61B 8/12, A61B 8/00, A61B 8/08

(54) **INTERLOCKING COMPONENTS FOR INTRALUMINAL ULTRASOUND IMAGING**
INEINANDERGREIFENDE KOMPONENTEN FÜR INTRALUMINALE ULTRASCHALLBILDGEBUNG
COMPOSANTS DE VERROUILLAGE POUR IMAGERIE ULTRASONORE INTRALUMINALE

(30) Priority: 27.02.2020 US 202062982266 P
(43) Date of publication of application: 04.01.2023
(73) Proprietor: Philips Image Guided Therapy Corporation, San Diego CA 92130 (US); Koninklijke Philips N.V., 5656 AG Eindhoven (NL)
(72) Inventor: MINAS, Maritess, 5656 AE Eindhoven (NL); WROLSTAD, David Kenneth, 5656 AE Eindhoven (NL); WILLIAMS, Nathan Andrew, 5656 AE Eindhoven (NL)
(74) Representative: Philips Intellectual Property & Standards
(86) International application number: PCT/EP2021/054241
(87) International publication number: WO 2021/170510

(56) References cited:
- WO-A1-2017/168300
- WO-A1-2019/110404
- WO-A1-2019/115568

## Description

### TECHNICAL FIELD

The present disclosure relates generally to intraluminal medical imaging, such as intravascular ultrasound (IVUS) imaging. In particular, projections and recesses can be provided on different components forming the distal structure of an intraluminal imaging device, such as an IVUS catheter, to increase the tensile strength of the connection between these components.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Solid-state (also known as synthetic-aperture) IVUS catheters are one of the two types of IVUS devices commonly used today, the other type being the rotational IVUS catheter. Solid-state IVUS catheters carry a scanner assembly that includes an array of ultrasound transducers distributed around its circumference along with one or more integrated circuit controller chips mounted adjacent to the transducer array. The controllers select individual acoustic elements (or groups of elements) for transmitting an ultrasound pulse and for receiving the ultrasound echo signal. By stepping through a sequence of transmit-receive pairs, the solid-state IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma. Furthermore, because there is no rotating element, the electrical interface is simplified. The solid-state scanner can be wired directly to the imaging system with a simple electrical cable and a standard detachable electrical connector, rather than the complex rotating electrical interface required for a rotational IVUS device.

Manufacturing solid-state IVUS devices sufficiently narrow to traverse anatomic structures within the human vasculature is challenging and two general performance standards must be met. First, IVUS devices must be extremely narrow to successfully pass through the small lumens in the human body without damaging tissue and second, despite their small size, intraluminal imaging devices must have high tensile strength to ensure that the device or parts of the device do not separate during a procedure. Such a breakage may cause parts of an intraluminal imaging device to be left within the heart or vasculature creating a condition that is extremely difficult to remedy. Generally, connections between various components of intraluminal imaging devices exhibit weaker tensile strength than unitary structures and are therefore more prone to separation. In addition, current methods of connecting components of intraluminal imaging devices often result in increased overall diameters of the device limiting the ability of the device to safely and successfully maneuver through constrained spaces. Increased overall diameter at connections may also make the surface of the device less uniform and more disposed to agitate or damage tissues within the body.

WO 2019/115568 A1 discloses an intraluminal imaging device comprising a flexible elongate member configured to be inserted into a body lumen of a patient, an ultrasound imaging assembly disposed at the distal portion of the flexible elongate member. The imaging assembly comprises a support member, a flexible substrate positioned around the support member, a plurality of ultrasound transducer elements integrated in the flexible substrate, and a plurality of control circuits disposed on the flexible substrate at a position proximal to the plurality of transducer elements. A distal member is coupled to the distal portion of the support member. The flexible substrate, the support member, the distal member, a proximal inner member, and/or a proximal outer member are coupled to one another via an adhesive.

WO 2017/168300 A1 discloses an intraluminal imaging device comprising a flexible elongate member configured to be inserted into a lumen of a patient, an ultrasound imaging assembly disposed at the distal portion of the a flexible elongate member and configured to obtain ultrasound imaging data while positioned within the lumen of the patient, a tip member disposed at the distal portion of the flexible elongate member, the tip member comprising a cavity adjacent to the ultrasound imaging assembly and configured to be filled with an adhesive to couple the tip member and the ultrasound imaging assembly.

### SUMMARY

The invention is defined by the claims. Embodiments of the present disclosure are intraluminal imaging devices, such as intravascular ultrasound (IVUS) catheters. The IVUS catheter described herein provides stronger attachment between components at the distal end, which overcome the limitations described above. For example, an ultrasound imaging assembly is coupled to a proximal end of the tip member. The imaging assembly is formed of a polymer support member and a flexible substrate positioned around the support member. The distal end of the support member includes one or more recesses. A proximal end of the tip member includes one or more protrusions extending inwardly into the inner lumen created by the tip member. These proximal protrusions received into the corresponding recesses of the support member. As a result, the proximal protrusions of the tip member directly contact an inner polymer member forming part of the catheter body and extending through the lumen created by the support member. When heated during a reflow process, the proximal protrusions of the polymer tip member fuse with the polymer inner member and form a strong mechanical connection in and around the support member. Adhesive may be used to further bond the tip member to the support member and flexible substrate. In other embodiments, the protrusions and recesses are located in different places, such as different components of the IVUS catheter than those described above. For example, the support member can include projections that are received within recesses of the tip member. Connecting the components of the IVUS catheter as described herein increase the tensile strength of these bonds while also ensuring a smaller overall diameter.

In an exemplary aspect, an intraluminal imaging device is provided. The intraluminal imaging device comprises a flexible elongate member configured to be positioned within a body lumen of a patient, the flexible elongate member comprising a proximal portion and a distal portion; an ultrasound imaging assembly disposed at the distal portion of the flexible elongate member and configured to obtain ultrasound data while positioned within the body lumen; and a tip member coupled to the ultrasound imaging assembly, wherein the tip member comprises a protrusion, wherein the ultrasound imaging assembly comprises a recess, and wherein the protrusion is received within the recess.

In some aspects, the ultrasound imaging assembly comprises a support member and a flexible substrate positioned around the support member, and the support member comprises the recess. In some aspects, the support member comprises a proximal portion and a distal portion, wherein the recess is disposed at the distal portion of the support member, the tip member comprises a proximal portion and a distal portion, the protrusion is disposed at the proximal portion of the tip member, and the distal portion of the support member is coupled to the proximal portion of the tip member. In some aspects, the flexible elongate member comprises an inner member extending through the support member such that the recess exposes a portion of the inner member. In some aspects, the support member comprises a first surface and an opposite, second surface, and the recess extends from the first surface to the second surface. In some aspects, the protrusion is in contact with the exposed portion of the inner member. In some aspects, the protrusion is coupled to the exposed portion of the inner member. In some aspects, the tip member comprises a first polymer and the inner member comprises a second polymer, and the first polymer of the protrusion is fused to the second polymer of the inner member. In some aspects, the protrusion extends radially inward. In some aspects, the support member comprises a plurality of recesses, and the tip member comprises a plurality of protrusions, wherein the plurality of protrusions is received within the plurality of recesses. In some aspects, the tip member comprises an outer surface, the outer surface comprises a first sloped portion and an opposite, second sloped portion, and the protrusion is aligned with the first sloped portion.

In an exemplary aspect, an intraluminal imaging device is provided. The intraluminal imaging device comprises a flexible elongate member configured to be positioned within a body lumen of a patient, the flexible elongate member comprising a proximal portion and a distal portion; an ultrasound imaging assembly disposed at the distal portion of the flexible elongate member and configured to obtain ultrasound data while positioned within the body lumen, wherein the ultrasound imaging assembly comprises: a support member comprising a first stand, a second stand, a third stand, and a ridge; and a flexible substrate positioned around the first stand, the second stand, and the third stand, wherein the flexible substrate comprises a plurality of integrated circuit chips and an ultrasound transducer assembly; a filling material between the first stand, the second stand, and a portion of the flexible substrate comprising the plurality of integrated circuit chips; and an acoustic backing material between the second stand, the third stand, and a portion of the flexible substrate comprising the ultrasound transducer assembly; and a tip member coupled to the ultrasound imaging assembly, wherein the tip member comprises a groove receiving the ridge.

In some aspects, the groove of the tip member is configured to engage the ridge of the support member to allow relative movement between the tip member and support member in a first direction and restrict the relative movement in an opposite, second direction. In some aspects, the groove of the tip member comprises a proximal portion and a distal portion, the proximal portion of the groove has a first diameter larger than a second diameter of the distal portion of the groove, the ridge of the support member comprises a proximal portion and a distal portion, and the proximal portion of the ridge has a third diameter larger than a fourth diameter of the distal portion of the ridge. In some aspects, the support member comprises a plurality of ridges, and the tip member comprises a plurality of grooves receiving the plurality of ridges.

In an exemplary aspect, an intravascular ultrasound (IVUS) imaging catheter is provided. The IVUS imaging catheter comprises a flexible elongate member configured to be positioned within a blood vessel of a patient, the flexible elongate member comprising a proximal portion, a distal portion, an inner polymer member, and an outer polymer member positioned around the inner polymer member; an ultrasound imaging assembly disposed at the distal portion of the flexible elongate member and configured to obtain ultrasound data while positioned within the blood vessel, wherein the ultrasound imaging assembly comprises a support member and a flexible substrate positioned around the support member, wherein the flexible substrate comprises an ultrasound transducer array, wherein the inner polymer member extends through the support member, wherein the support member comprises a plurality of recesses exposing portions of the inner member extending through support member; and a polymer tip member coupled to the ultrasound imaging assembly, wherein the polymer tip member comprises a plurality of protrusions received within the plurality of recesses such that the plurality of protrusions is fused to the exposed portions of the polymer inner member.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a diagrammatic schematic view of an intraluminal imaging system.
Fig. 2 is a diagrammatic top view of a ultrasound imaging assembly in a flat configuration.
Fig. 3 is a diagrammatic perspective view of the ultrasound imaging assembly shown in Fig. 2 in a rolled configuration around a support member.
Fig. 4 is a diagrammatic cross-sectional side view of the ultrasound imaging assembly shown in Fig. 3.
Fig. 5 is a side view of a support member, according to aspects of the present disclosure.
Fig. 6 is a diagrammatic cross-sectional view of the distal end of the intraluminal imaging device before a tip member is coupled to the intraluminal imaging device, according to aspects of the present disclosure.
Fig. 7 is a diagrammatic cross-sectional view of a tip member, according to aspects of the present disclosure.
Fig. 8 is a diagrammatic cross-sectional view of the connection of the distal end of the intraluminal imaging device shown in Fig. 6 and the proximal end of the tip member shown in Fig. 7, according to aspects of the present disclosure.
Fig. 9 is a partial cutaway view of a connection of the distal end of a support member and the proximal end of a tip member, according to aspects of the present disclosure.
Fig. 10 is a flow diagram of a method of assembling an intraluminal imaging device, according to aspects of the present disclosure.
Fig. 11 is a side view of a scanner assembly and a tip member positioned around an assembly mandrel before coupling the tip member to the scanner assembly, according to aspects of the present disclosure.
Fig. 12 is a side view of a scanner assembly and a tip member positioned around an assembly mandrel after coupling the tip member to the intraluminal imaging device, according to aspects of the present disclosure.
Fig. 13 is a side view of a heat shrink layer positioned around a scanner assembly and a tip member while supported by an assembly mandrel, according to aspects of the present disclosure.
Fig. 14 is a side view of an intraluminal imaging device after assembly, according to aspects of the present disclosure.
Fig. 15 is a diagrammatic cross-sectional view of an embodiment of the connection between components at the distal portion of the intraluminal imaging device, according to aspects of the present disclosure.
Fig. 16 is a diagrammatic cross-sectional view of an embodiment of the connection between components at the distal portion of the intraluminal imaging device, according to aspects of the present disclosure.
Fig. 17 is a diagrammatic cross-sectional view of an embodiment of the connection between components at the distal portion of the intraluminal imaging device, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. Any alterations and further modifications to the described devices, systems, and methods, and any further application of the principles of the present disclosure are fully contemplated and included within the present disclosure as would normally occur to one skilled in the art to which the disclosure relates. For example, while the focusing system is described in terms of cardiovascular imaging, it is understood that it is not intended to be limited to this application. The system is equally well suited to any application requiring imaging within a confined cavity. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

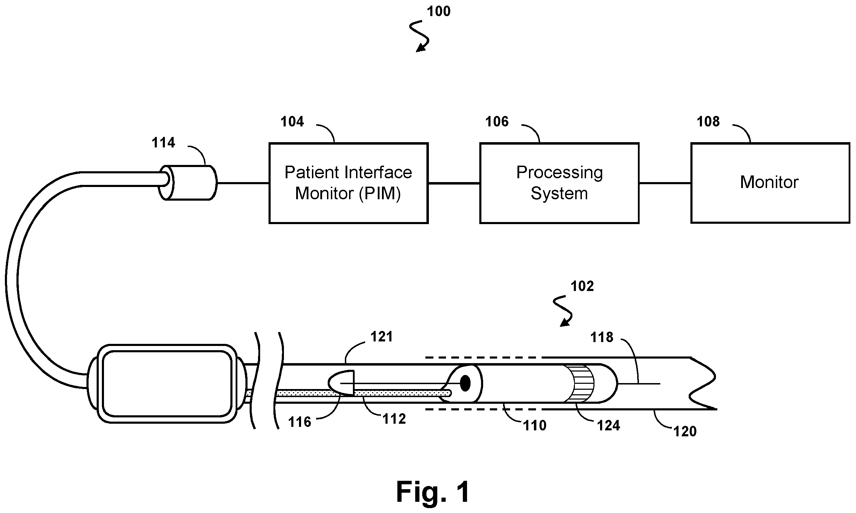

Fig. 1 is a diagrammatic schematic view of an intraluminal imaging system 100. The intraluminal imaging system 100 can be an ultrasound imaging system. In some instances, the system 100 can be an intravascular ultrasound (IVUS) imaging system. The system 100 may include an intraluminal imaging device 102 such as a catheter, guide wire, or guide catheter, a patient interface module (PIM) 104, a processing system or console 106, and a monitor 108. The intraluminal imaging device 102 can be an ultrasound imaging device. In some instances, the device 102 can be an IVUS imaging device, such as a solid-state IVUS device.

At a high level, the IVUS device 102 emits ultrasonic energy from a transducer array 124 included in scanner assembly 110 mounted near a distal end of the catheter device. The ultrasonic energy is reflected by tissue structures in the medium, such as a vessel 120, or another body lumen surrounding the scanner assembly 110, and the ultrasound echo signals are received by the transducer array 124. In that regard, the device 102 can be sized, shaped, or otherwise configured to be positioned within the body lumen of a patient. The PIM 104 transfers the received echo signals to the console or computer 106 where the ultrasound image (including the flow information) is reconstructed and displayed on the monitor 108. The console or computer 106 can include a processor and a memory. The computer or computing device 106 can be operable to facilitate the features of the IVUS imaging system 100 described herein. For example, the processor can execute computer readable instructions stored on the non-transitory tangible computer readable medium.

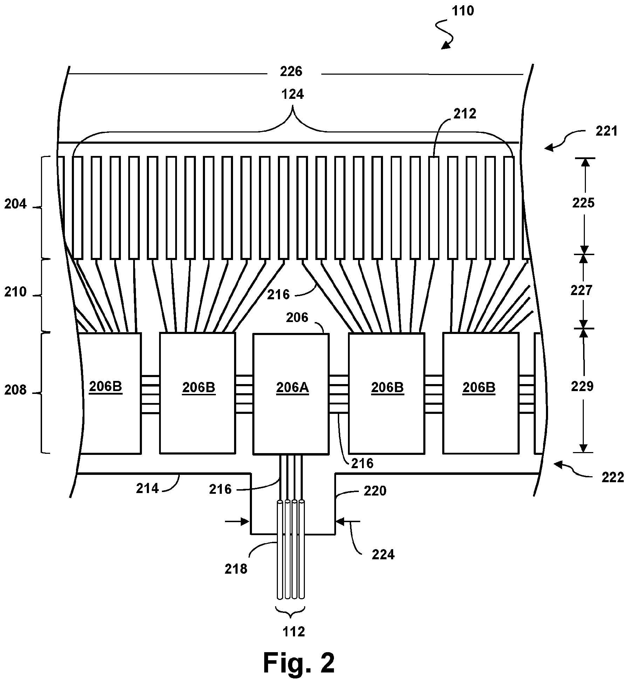

The PIM 104 facilitates communication of signals between the IVUS console 106 and the scanner assembly 110 included in the IVUS device 102. This communication includes the steps of: (1) providing commands to integrated circuit controller chip(s) 206A and 206B, illustrated in Fig. 2, included in the scanner assembly 110 to select the particular transducer array element(s), or acoustic element(s), to be used for transmit and receive, (2) providing the transmit trigger signals to the integrated circuit controller chip(s) 206A and 206B included in the scanner assembly 110 to activate the transmitter circuitry to generate an electrical pulse to excite the selected transducer array element(s), and/or (3) accepting amplified echo signals received from the selected transducer array element(s) via amplifiers included on the integrated circuit controller chip(s)126 of the scanner assembly 110. In some embodiments, the PIM 104 performs preliminary processing of the echo data prior to relaying the data to the console 106. In examples of such embodiments, the PIM 104 performs amplification, filtering, and/or aggregating of the data. In an embodiment, the PIM 104 also supplies high- and low-voltage DC power to support operation of the device 102 including circuitry within the scanner assembly 110.

The IVUS console 106 receives the echo data from the scanner assembly 110 by way of the PIM 104 and processes the data to reconstruct an image of the tissue structures in the medium surrounding the scanner assembly 110. The console 106 outputs image data such that an image of the vessel 120, such as a cross-sectional image of the vessel 120, is displayed on the monitor 108. Vessel 120 may represent fluid filled or surrounded structures, both natural and man-made. The vessel 120 may be within a body of a patient. The vessel 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

In some embodiments, the IVUS device includes some features similar to traditional solid-state IVUS catheters, such as the EagleEye^{®} catheter available from Volcano Corporation and those disclosed in U.S. Patent No. 7,846,101. For example, the IVUS device 102 includes the scanner assembly 110 near a distal end of the device 102 and a transmission line bundle 112 extending along the longitudinal body of the device 102. The transmission line bundle or cable 112 can include a plurality of conductors, including one, two, three, four, five, six, seven, or more conductors 218 (Fig. 2). It is understood that any suitable gauge wire can be used for the conductors 218. In an embodiment, the cable 112 can include a four-conductor transmission line arrangement with, e.g., 0.0711 mm diameter (41 AWG gauge) wires. In an embodiment, the cable 112 can include a seven-conductor transmission line arrangement utilizing, e.g., 0.0508 mm diameter (44 AWG gauge) wires. In some embodiments, 0.0559 mm diameter (43 AWG gauge) wires can be used.

The transmission line bundle 112 terminates in a PIM connector 114 at a proximal end of the device 102. The PIM connector 114 electrically couples the transmission line bundle 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

Fig. 2 is a diagrammatic top view of a portion of a flexible assembly 110. The flexible assembly 110 includes a transducer array 124 formed in a transducer region 204 and transducer control logic dies 206 (including dies 206A and 206B) formed in a control region 208, with a transition region 210 disposed therebetween. The transducer array 124 includes an array of ultrasound transducers 212. The transducer control logic dies 206 are mounted on a flexible substrate 214 into which the transducers 212 have been previously integrated. The flexible substrate 214 is shown in a flat configuration in Fig. 2. Though six control logic dies 206 are shown in Fig. 2, any number of control logic dies 206 may be used. For example, one, two, three, four, five, six, seven, eight, nine, ten, or more control logic dies 206 may be used.

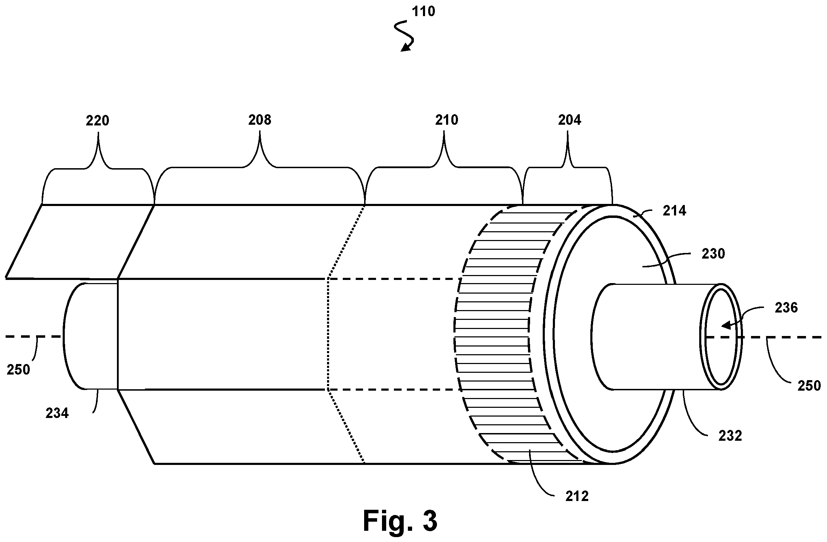

The flexible substrate 214, on which the transducer control logic dies 206 and the transducers 212 are mounted, provides structural support and interconnects for electrical coupling. The flexible substrate 214 may be constructed to include a film layer of a flexible polyimide material such as KAPTON^{™} (trademark of DuPont). Other suitable materials include polyester films, polyimide films, polyethylene napthalate films, or polyetherimide films, liquid crystal polymer, other flexible printed semiconductor substrates as well as products such as Upilex^{®} (registered trademark of Ube Industries) and TEFLON^{®} (registered trademark of E.I. du Pont). In the flat configuration illustrated in Fig. 2, the flexible substrate 214 has a generally rectangular shape. As shown and described herein, the flexible substrate 214 is configured to be wrapped around a support member 230 (Fig. 3) in some instances. Therefore, the thickness of the film layer of the flexible substrate 214 is generally related to the degree of curvature in the final assembled flexible assembly 110. In some embodiments, the film layer is between 5 µm and 100 µm, with some particular embodiments being between 5 µm and 25.1 µm, e.g., 6 µm.

The set of transducer control logic dies 206 is a non-limiting example of a control circuit. The transducer region 204 is disposed at a distal portion 221 of the flexible substrate 214. The control region 208 is disposed at a proximal portion 222 of the flexible substrate 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or, the length 227 of the transition region 210 may be less than lengths 225 and 229, the length 227 of the transition region 210 can be greater than lengths 225, 229 of the transducer region and controller region, respectively.

The control logic dies 206 are not necessarily homogenous. In some embodiments, a single controller is designated a master control logic die 206A and contains the communication interface for cable 112, between a processing system, e.g., processing system 106, and the flexible assembly 110. Accordingly, the master control circuit may include control logic that decodes control signals received over the cable 112, transmits control responses over the cable 112, amplifies echo signals, and/or transmits the echo signals over the cable 112. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

To electrically interconnect the control logic dies 206 and the transducers 212, in an embodiment, the flexible substrate 214 includes conductive traces 216 formed in the film layer that carry signals between the control logic dies 206 and the transducers 212. In particular, the conductive traces 216 providing communication between the control logic dies 206 and the transducers 212 extend along the flexible substrate 214 within the transition region 210. In some instances, the conductive traces 216 can also facilitate electrical communication between the master controller 206A and the slave controllers 206B. The conductive traces 216 can also provide a set of conductive pads that contact the conductors 218 of cable 112 when the conductors 218 of the cable 112 are mechanically and electrically coupled to the flexible substrate 214. Suitable materials for the conductive traces 216 include copper, gold, aluminum, silver, tantalum, nickel, and tin, and may be deposited on the flexible substrate 214 by processes such as sputtering, plating, and etching. In an embodiment, the flexible substrate 214 includes a chromium adhesion layer. The width and thickness of the conductive traces 216 are selected to provide proper conductivity and resilience when the flexible substrate 214 is rolled. In that regard, an exemplary range for the thickness of a conductive trace 216 and/or conductive pad is between 1-5 µm. For example, in an embodiment, 5 µm conductive traces 216 are separated by 5 µm of space. The width of a conductive trace 216 on the flexible substrate may be further determined by the width of the conductor 218 to be coupled to the trace/pad.

The flexible substrate 214 can include a conductor interface 220 in some embodiments. The conductor interface 220 can be in a location of the flexible substrate 214 where the conductors 218 of the cable 112 are coupled to the flexible substrate 214. For example, the bare conductors of the cable 112 are electrically coupled to the flexible substrate 214 at the conductor interface 220. The conductor interface 220 can be tab extending from the main body of flexible substrate 214. In that regard, the main body of the flexible substrate 214 can refer collectively to the transducer region 204, controller region 208, and the transition region 210. In the illustrated embodiment, the conductor interface 220 extends from the proximal portion 222 of the flexible substrate 214. In other embodiments, the conductor interface 220 is positioned at other parts of the flexible substrate 214, such as the distal portion 221, or the flexible substrate 214 may lack the conductor interface 220. A value of a dimension of the tab or conductor interface 220, such as a width 224, can be less than the value of a dimension of the main body of the flexible substrate 214, such as a width 226. In some embodiments, the substrate forming the conductor interface 220 is made of the same material(s) and/or is similarly flexible as the flexible substrate 214. In other embodiments, the conductor interface 220 is made of different materials and/or is comparatively more rigid than the flexible substrate 214. For example, the conductor interface 220 can be made of a plastic, thermoplastic, polymer, hard polymer, etc., including polyoxymethylene (e.g., DELRIN^{®}), polyether ether ketone (PEEK), nylon, Liquid Crystal Polymer (LCP), and/or other suitable materials.

Fig. 3 illustrates a perspective view of the scanner assembly 110 in a rolled configuration. In some instances, the flexible substrate 214 is transitioned from a flat configuration (Fig. 2) to a rolled or more cylindrical configuration (Fig. 3). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND SENSING ASSEMBLY HAVING A FLEXIBLE SUBSTRATE".

In some embodiments, the transducer elements 212 and/or the controllers 206 can be positioned in an annular configuration, such as a circular configuration or in a polygon configuration, around a longitudinal axis 250 of a support member 230. It will be understood that the longitudinal axis 250 of the support member 230 may also be referred to as the longitudinal axis of the scanner assembly 110, the flexible elongate member 121, and/or the device 102. For example, a cross-sectional profile of the imaging assembly 110 at the transducer elements 212 and/or the controllers 206 can be a circle or a polygon. Any suitable annular polygon shape can be implemented, such as one based on the number of controllers/transducers, flexibility of the controllers/transducers, etc., including a pentagon, hexagon, heptagon, octagon, nonagon, decagon, etc. In some examples, the plurality of transducer controllers 206 may be used for controlling the plurality of ultrasound transducer elements 212 to obtain imaging data associated with the vessel 120.

The support member 230 can be referenced as a unibody in some instances. The support member 230 can be composed of a metallic material, such as stainless steel, or a non-metallic material, such as a plastic or polymer as described in U.S. Provisional Application No. 61/985,220, "Pre-Doped Solid Substrate for Intravascular Devices," filed April 28, 2014, ('220 Application). In some embodiments, support member 230 may be composed of 303 stainless steel. The support member 230 can be a ferrule having a distal flange or portion 232 and a proximal flange or portion 234. The support member 230 can be tubular in shape and define a lumen 236 extending longitudinally therethrough. The lumen 236 can be sized and shaped to receive the guide wire 118. The support member 230 can be manufactured using any suitable process. For example, the support member 230 can be machined and/or electrochemically machined or laser milled, such as by removing material from a blank to shape the support member 230, or molded, such as by an injection molding process.

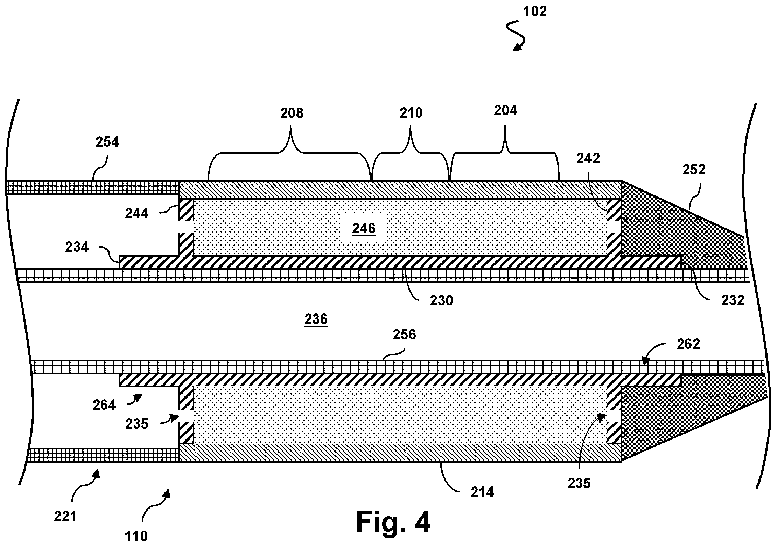

Referring now to Fig. 4, shown there is a diagrammatic cross-sectional side view of a distal portion of the intraluminal imaging device 102, including the flexible substrate 214 and the support member 230. The support member 230 can be referenced as a unibody in some instances. The support member 230 can be composed of a metallic material, such as stainless steel, or a non-metallic material, such as a plastic or polymer as described in U.S. Provisional Application No. 61/985,220, "Pre-Doped Solid Substrate for Intravascular Devices," filed April 28, 2014. The support member 230 can be ferrule having a distal portion 262 and a proximal portion 264. The support member 230 can define a lumen 236 extending along the longitudinal axis LA. The lumen 236 is in communication with the entry/exit port 116 and is sized and shaped to receive the guide wire 118 (Fig. 1). The support member 230 can be manufactured according to any suitable process. For example, the support member 230 can be machined and/or electrochemically machined or laser milled, such as by removing material from a blank to shape the support member 230, or molded, such as by an injection molding process. In some embodiments, the support member 230 may be integrally formed as a unitary structure, while in other embodiments the support member 230 may be formed of different components, such as a ferrule and stands 242, 244, that are fixedly coupled to one another. In some cases, the support member 230 and/or one or more components thereof may be completely integrated with inner member 256. In some cases, the inner member 256 and the support member 230 may be joined as one, e.g., in the case of a polymer support member.

Stands 242, 244 that extend vertically are provided at the distal and proximal portions 262, 264, respectively, of the support member 230. The stands 242, 244 elevate and support the distal and proximal portions of the flexible substrate 214. In that regard, portions of the flexible substrate 214, such as the transducer portion 204 (or transducer region 204), can be spaced from a central body portion of the support member 230 extending between the stands 242, 244. The stands 242, 244 can have the same outer diameter or different outer diameters. For example, the distal stand 242 can have a larger or smaller outer diameter than the proximal stand 244 and can also have special features for rotational alignment as well as control chip placement and connection. To improve acoustic performance, any cavities between the flexible substrate 214 and the surface of the support member 230 are filled with a backing material 246. The liquid backing material 246 can be introduced between the flexible substrate 214 and the support member 230 via passageways 235 in the stands 242, 244, or through additional recesses as will be discussed in more detail hereafter. In some embodiments, suction can be applied via the passageways 235 of one of the stands 242, 244, while the liquid backing material 246 is fed between the flexible substrate 214 and the support member 230 via the passageways 235 of the other of the stands 242, 244. The backing material can be cured to allow it to solidify and set. In various embodiments, the support member 230 includes more than two stands 242, 244, only one of the stands 242, 244, or neither of the stands. In that regard the support member 230 can have an increased diameter distal portion 262 and/or increased diameter proximal portion 264 that is sized and shaped to elevate and support the distal and/or proximal portions of the flexible substrate 214.

The support member 230 can be substantially cylindrical in some embodiments. Other shapes of the support member 230 are also contemplated including geometrical, non-geometrical, symmetrical, non-symmetrical, cross-sectional profiles. As the term is used herein, the shape of the support member 230 may reference a cross-sectional profile of the support member 230. Different portions of the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can comprise a flexible elongate member. The proximal inner member 256 can be received within a proximal flange 234. The proximal outer member 254 abuts and is in contact with the proximal end of flexible substrate 214. A distal tip member 252 is coupled to the distal portion 262 of the support member 230. For example, the distal member 252 is positioned around the distal flange 232. The tip member 252 can abut and be in contact with the distal end of flexible substrate 214 and the stand 242. In other embodiments, the proximal end of the tip member 252 may be received within the distal end of the flexible substrate 214 in its rolled configuration. In some embodiments there may be a gap between the flexible substrate 214 and the tip member 252. The distal member 252 can be the distal-most component of the intraluminal imaging device 102.

One or more adhesives can be disposed between various components at the distal portion of the intraluminal imaging device 102. For example, one or more of the flexible substrate 214, the support member 230, the distal member 252, the proximal inner member 256, and/or the proximal outer member 254 can be coupled to one another via an adhesive.

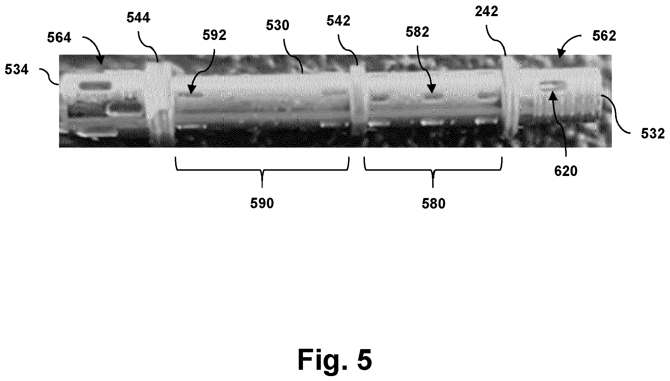

Fig. 5 is a side view of an embodiment of a support member 530, according to aspects of the present disclosure. Support member 530 may exhibit similar characteristics as support member 230 shown in Fig. 4. For example, the support member 530 can be referenced as a unibody in some instances. The support member 530 may also be composed of a metallic material, such as stainless steel, gold, silver, copper, and/or alloy, or a non-metallic material, such as a plastic or polymer as previously described in relation to support member 230. The support member 530 can be ferrule having a distal portion 562 and a proximal portion 564. Support member 530 may also comprise a distal flange or end 532 and proximal flange or end 534. The support member 530 may also be manufactured according to any suitable process. These processes may be substantially similar to manufacturing processes of support member 230. Support member 530 may comprise one or more stands extending radially from support member 530. These stands may be substantially similar to stands 242 and 244 and may serve similar functions. Support member 530 may comprise additional or fewer stands depending on the specific application of a particular embodiment. Support member 530 may be of any suitable shape. As shown in Fig. 5, one embodiment of support member 530 may be generally cylindrical in shape. Other shapes of the support member 530 are fully contemplated including geometrical, non-geometrical, symmetrical, and non-symmetrical cross-sectional profiles. Support member 530 may be of any suitable size. For example, support member 530 may be of sufficiently small diameter to maneuver through the coronary vasculature surrounding the human heart. In some embodiments, the support member 530 can be relatively larger for peripheral applications, to maneuver through vasculature in the legs, abdomen, neck, and other anatomies of the human body. In other embodiments in which the intraluminal imaging device is to be used to image still larger lumens, either within organic or inorganic lumens, the overall dimensions of support member 530 may be adjusted and increased accordingly.

Also depicted in Fig. 5 are multiple stands in addition to stand 242. As previously mentioned, support member 530 may comprise any number of stands of any suitable shape extending in an outward direction generally radially from the support member ferrule. In some embodiments, stand 242 may be positioned at a location near the distal end 532 of support member 530 and support member 530 may additionally comprise a proximal stand 544 positioned near the proximal end 534 of support member with an intermediate stand 542 positioned therebetween. These three stands, the distal stand 242, the intermediate stand 542, and the proximal stand 544, may serve various purposes or functions within ultrasound imaging assembly 110. One purpose of distal stand 242, intermediate stand 542, and proximal stand 544 may be to provide support for flexible substrate 214. In addition, intermediate stand 542 may be placed at a location proximal to the transducers 212 disposed on the surface of flexible substrate 214.

Distal stand 242 and intermediate stand 542 define a region 580 extending longitudinally along the support member 530. Transducers 212 on flexible substrate 214 may be positioned over region 580 such that transducers 212 are situated generally at the same longitudinal position as region 580 and radially outward from region 580. The ferrule surface of support member 530 at region 580 between distal stand 242 and intermediate stand 542 may comprise one or more recesses 582. Recesses 582 may be configured to extend completely through the exterior of support member 530 to the interior or inner lumen 236 within support member 530. Recesses 582 may thereby allow direct access to the inner lumen 236 of support member 530 from the exterior environment of support member 530 and vice versa. One function of recesses 582 may be to introduce acoustic backing material 246 (Fig. 4) to be positioned within the lumen created by distal stand 242, intermediate stand 542, region 580 and the inner surface of flexible substrate 214. Acoustic backing material 246 may be used to attenuate signals which may propagate from transducers 212 of flexible substrate 214 in an inwardly radial direction. Acoustic backing material 246 may also attenuate reflected waves received by transducers 212 which may propagate from a source radially inward of transducers 212. Due to these acoustic properties, acoustic backing material is positioned radially inward of transducers 212. Intermediate stand 542 may serve as a barrier between region 580 filled with acoustic backing material 246 and region 590 which may not need to be filled with acoustic backing material 246.

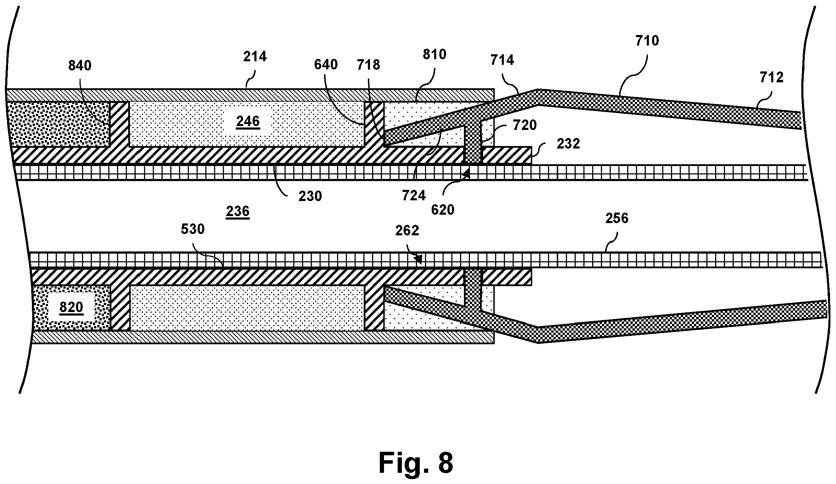

Intermediate stand 542 and proximal stand 544 may define region 590 extending longitudinally along the support member 530. Integrated circuit controller chip(s) 206A and 206B may be positioned over region 590 such that integrated circuit controller chip(s) 206A and 206B are situated generally at the same longitudinal position as region 590 and radially outward from region 590. The ferrule surface of support member 530 at region 590 between intermediate stand 542 and proximal stand 544 may comprise one or more recesses 592. Recesses 592 may be configured to extend completely through the exterior of support member 530 to the interior or inner lumen 236 within support member 530. Recesses 592 may thereby allow direct access to the inner lumen 236 of support member 530 from the exterior environment of support member 530 and vice versa. One function of recesses 592 may be to introduce filling material 820 (Fig. 8) to be positioned within the lumen created by intermediate stand 542, proximal stand 544, region 590 and the inner surface of flexible substrate 214. Region 590 may be composed of any suitable metallic or non-metallic material. In some embodiments, region 590 may be filled with an underfill material to the support the chips 206A and 206B. The underfill material can be an adhesive, an epoxy, etc. In some embodiments, the underfill material need not have acoustic attenuating properties as the acoustic backing material 246 because the underfill material is under the chips 206A, 206B, and not the transducers 212. Filling material 820 may serve to physically support flexible substrate 214 generally around region 590. Filling material 820 may be positioned at a location radially inward of integrated circuit controller chip(s) 206A and 206B as well as other components previously mentioned that may be positioned on the surface of flexible substrate 214.

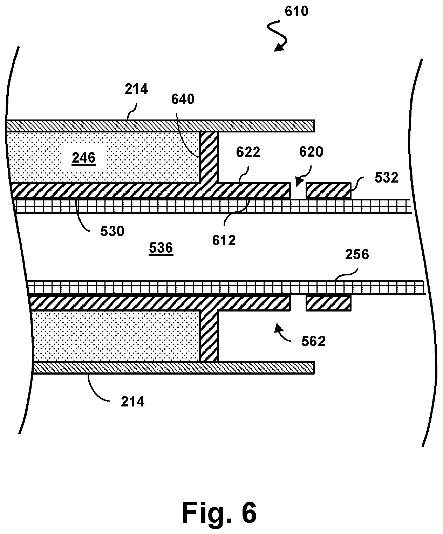

Also depicted in Fig. 5 is recess 620 positioned near the distal end 532 of support member 530. As will be discussed in reference to Fig. 6, recess 620 may extend completely through the exterior of support member 530 to the interior or inner lumen 236 within support member 530. Recess 620 may thereby allow direct access to the inner lumen 236 of support member 530 from the exterior environment of support member 530 and vice versa.

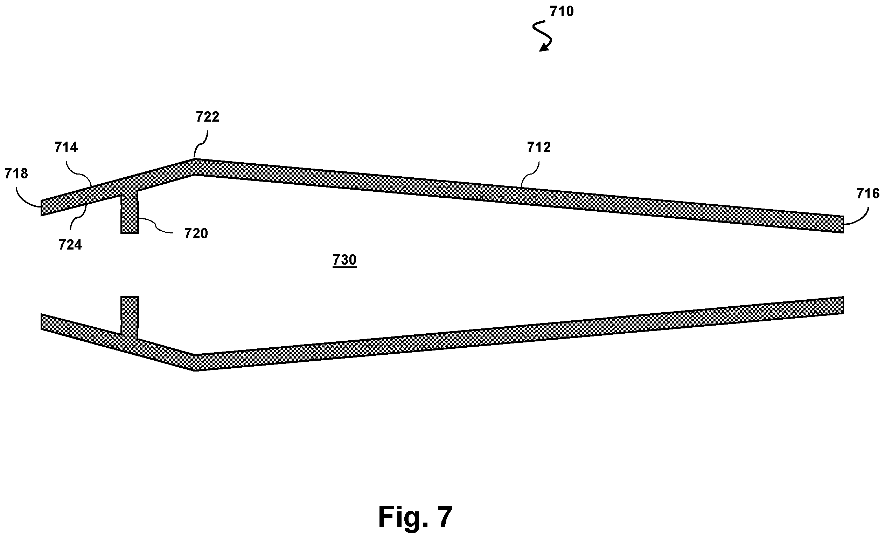

Fig. 6 is a diagrammatic cross-sectional view of the distal portion of an ultrasound imaging assembly 610 before a tip member 710 (Fig. 7) is coupled to the ultrasound imaging assembly 610, according to aspects of the present disclosure. Ultrasound imaging assembly 610 may be substantially similar to ultrasound imaging assembly 110 previously presented or may differ. As shown in Fig. 6, ultrasound imaging assembly 610 may comprise support member 530. Support member 530 can be tubular in shape and define a lumen 536 extending longitudinally therethrough. The lumen 536 can be sized and shaped to receive the guide wire 118. Lumen 536 may further be configured to receive inner member 256 therethrough.

Support member 530 may further comprise a stand 640. Stand 640 may extend radially from support member 530. Stand 640 may also be referred to as a lip or shoulder. Stand 640 and support member 530 may be integrally formed as a unitary structure, while in other embodiments, stand 640 may be formed of a different component than support member 530. Stand 640 may be fixedly coupled to support member 530. In different embodiments, stand 640 may be manufactured of different materials than support member 530, or the same materials. In any case, stand 640 may be manufactured of any suitable material, including metallic and non-metallic materials depending on the specific application of a particular embodiment. Stand 640 may be of any suitable shape. For example, stand 640 may be generally circular extending completely around and in direct contact with support member 530. In other embodiments, stand 640 may be replaced with multiple components spaced a distance apart from one another and positioned at the same longitudinal position around support member 530. In addition, stand 640 may extend any suitable distance from support member 530.

Ultrasound imaging assembly 610 may further comprise flexible substrate 214. Flexible substrate 214 may be fixedly coupled to the outer surface of stand 640 in such a way that stand 640 may support flexible substrate 214. Flexible substrate 214 may thereby be positioned at an appropriate space away from support member 530 so as to create a gap between these two components. This gap is created by stands which may be substantially similar to stand 640, stand 242, stand 542, or stand 544 which extend radially outward from the ferrule of support member 530 in such a way that the outer diameter of the stands is greater than the overall diameter of the support member 530 ferrule. This gap may be filled with any suitable materials or components, including but not limited to an acoustic backing material 246, filling material 820 (Fig. 8), other attenuating material, or any suitable material. In the particular embodiment shown in Fig. 6, the gap between flexible substrate 214 and support member 530 as defined by stand 640 is filled with acoustic backing material 246. To improve acoustic performance, any cavities between the flexible substrate 214 and the surface of the support member 230 are generally filled with a backing material 246. The liquid backing material 246 has a relatively low acoustic impedance, and can be introduced between the flexible substrate 214 and the support member 230 via passageways in the support member 230 (not shown). Backing material 246 may be used to attenuate or absorb acoustic energy not directed to the anatomy of interest. The backing material 246 fills the space between the support member 230 and the transducer array 124 as well as the gaps between adjacent individual transducers 212. The backing material 246 possesses the ability to highly attenuate the ultrasound which is transmitted by the transducer array 124. The backing material 246 also provides support for the transducer elements. The backing material 246 can be cured to allow it to solidify and set in a sufficiently short period of time to meet manufacturing needs. A number of known materials meeting the above described criteria for a good backing material will be known to those skilled in the art. An example of such a backing material comprises a mixture of epoxy, hardener and phenolic microballoons providing high ultrasound signal attenuation and satisfactory support for the ultrasound transducer assembly. Still other materials for baking material 246 may include polymers, graphite, composites, ceramics, metals, or any combination thereof.

In some embodiments, positioned at the distal portion 562 of support member 530, support member 530 may comprise a plurality of recesses 620. In its cross-sectional view, support member 530 is pictured comprising two such recesses 620. However, support member 530 may comprise only one, three, four, or more recesses 620. In an embodiment, recess 620 may extend completely through the distal portion 562 of support member 530. For example, recess 620 may extend from surface 622 of recess 620 completely through a portion of support member 530 to the opposing surface 612 of support member 530. In this configuration, recess 620 allows for direct access to or exposes inner member 256. In other embodiments, however, recess 620 may not extend completely through support member 530 such that recess 620 does not expose inner member 256. In such an embodiment, recess 620 may only be configured on surface 622 of support member 530. Opposing surface 612 would not comprise any such recess, but would be substantially uniform. In still other embodiments, support member 530 may comprise some recesses which extend completely through support member 530, and some recesses which do not extend completely through support member 530. Recess 620 may be of any suitable size or shape. In some embodiments, recess 620 is of a generally circular shape. In other embodiments, the shape of recess 620 may be that of a rectangle, square, triangle, or any other polygon. Other shapes are also contemplated including geometrical, non-geometrical, symmetrical and non-symmetrical shapes. Recess 620 may also be referred to as a hole or cavity.

Fig. 7 is a diagrammatic cross-sectional view of a tip member 710, according to aspects of the present disclosure. Tip member 710 may also be referred to as a molded tip or lead tip. Tip member 710 may be constructed of any suitable material including a non-metallic material, such as a plastic or polymer. In some embodiments, tip member 710 is the leading component of intraluminal imaging device 602 while the device travels through a body lumen. Tip member 710 may be composed of any appropriate material, but may be flexible so as to not penetrate the body lumen. Tip member 710 may comprise a distal end 716 and a proximal end 718. Tip member 710 may also be of a generally conical shape. However, tip member 710 may be of any suitable shape configured to allow an intraluminal imaging device to easily maneuver through the vasculature of a patient. In addition, tip member 710 may be of any suitable size configured to accomplish the desired result in any particular application. The overall diameter of tip member 710 at a distal end 716 may be smaller than the overall diameter of tip member 710 at a proximal end 718. In addition, the overall diameter of tip member 710 at a proximal point 718 may be smaller than the overall diameter of tip member 710 at a point 722 positioned between distal end 716 and proximal end 718. Tip member 710 may then further comprise two sloped or tapered surfaces, surface 712 and surface 714 sloping in opposite directions from one another. In other words, surface 712 and surface 714 comprise opposite tapers. For example, surface 712 and surface 714 may extend towards the radial center of tip member 710 and away from point 722. Point 722 also need not be a singular longitudinal point along tip member 710. In other embodiments, there may be a larger region of substantially the same outer diameter in place of point 722. In still other embodiments, tip member 710 may not comprise surface 714. For example, the overall diameter of proximal end 718 may be the largest overall diameter at any longitudinal location of tip member 710 making tip member 710 a purely conical shape.

Tip member 710 may further define an inner lumen 730. Lumen 730 may comprise a space between the outer surfaces of tip member 710 and may be configured to receive inner member 256. The overall diameter of inner lumen 730 may be substantially similar to the diameter of inner member 256 or may be larger depending on the application so as to allow additional components to be received within lumen 730.

Tip member 710 may further comprise a plurality of protrusions 720 which extend from an inner surface 724 of tip member 710 in an inward direction towards the radial center of tip member 710 and protruding into lumen 730. In other embodiments, tip member 710 may comprise only one such protrusion 720, or may comprise two, three, four, or more protrusions 720. The dimensions of protrusion 720 may be of any suitable dimension and may be configured to correspond to or be received by recesses 620 of support member 530. In an embodiment, protrusion 720 may have a generally circular cross-sectional profile shape. In still other embodiments, it is fully contemplated that cross-sectional profiles of a rectangle, square, triangle, other polygon, and geometrical, non-geometrical, symmetrical and non-symmetrical shapes may be used for protrusion 720. In addition, though Fig. 7 presents protrusion 720 extending in a direction directly towards the radial center of tip member 710, in other embodiments, protrusion 720 may extend at any suitable angle, e.g. an oblique angle or the like, in an inward, radial direction. In addition, the overall width of protrusion 720 need not be uniform. In addition, protrusion 720 and tip member 710 may be integrally formed as a unitary structure, while in other embodiments protrusion 720 and tip member 710 may be formed of different components. As such, protrusion 720 may be constructed of a different material than tip member 710 or may be constructed of the same material.

Fig. 8 is a diagrammatic cross-sectional view of the connection of the distal end of the ultrasound imaging assembly 610 shown in Fig. 6 and the proximal end of the tip member 710 shown in Fig. 7, according to aspects of the present disclosure. The diameter of tip member 710 at proximal end 718 may be smaller than the overall diameter of the lumen created by flexible substrate 214 in its rolled configuration, such that the proximal end 718 may be received inside the distal portion of flexible substrate 214 in its rolled configuration, as shown in Fig. 8. In this configuration and as also shown in Fig. 8, the distal end or flange 232 of support member 530 is received within lumen 730 (Fig. 7) of tip member 710. The proximal end 718 of tip member 710 may be in direct contact with and abut stand 640 of support member 530. In other embodiments, proximal end 718 of tip member 710 may not abut stand 640 but may positioned at a location distal of stand 640 so as to create a gap.

When tip member 710 is placed in connection or direct contact with ultrasound imaging assembly 610, protrusions 720 of tip member 710 may be received into corresponding recesses 620 of support member 530. Protrusions 720 may be of the same cross-sectional profile shape as recesses 620 or may be of a different cross-sectional profile shape. Though Fig. 8 depicts two such protrusions 720 and two corresponding recesses 620, there may be more or fewer protrusions 720 and corresponding recesses 620. In addition, though both protrusions 720 and recesses 620 depicted in Fig. 8 are positioned at the same longitudinal location along ultrasound imaging assembly 610, this need not be the case. For example, in other embodiments, a protrusion 720 located on tip member 710 and a corresponding recess 620 located on support member 530 may be located at a point proximal or distal to another protrusion 720 and corresponding recess 620.

In some embodiments, protrusion 720 may extend completely through recess 620 such that the distal end of protrusion 720 is in direct contact with the outer surface of inner member 256. Tip member 710, including protrusion 720 may also be of the same material as inner member 256. This embodiment provides a strong mechanical bond between support member 530 and tip member 710. As previously stated, in other embodiments, recess 620 may not extend completely through support member 530 from surface 622 to surface 612. In these embodiments, recess 620 may only be positioned on surface 622 and surface 612 may be substantially uniform. In these embodiments, the proximal end of protrusion 720 would not extend completely through support member 530 and would not be in direct contact with the outer surface of inner member 256. A mechanical bond would still be formed between support member 530 and tip member 710 in this embodiment.

In some embodiments, a protrusion may be positioned at the distal region of support member 530 and a corresponding recess may be positioned on tip member 710. In such an embodiment, the protrusion may be of similar geometry to protrusion 720. However, the protrusion may extend radially outward from the ferrule of support member 530 and may be of the same material as support member 530 such that the protrusion and support member 530 are one unitary structure. The protrusion may also be of a different material. The protrusion may be positioned on surface 622 of support member 530. A recess positioned on the tip member 710 may receive the protrusion.

In an embodiment in which protrusion 720 extends completely through support member 530 and is direct contact with inner member 256, ultrasound imaging assembly 610 may be subject to a reflow process after assembly. This reflow process may be substantially similar to reflow processes known and readily available to those skilled in the art. During such a reflow process, the temperature of the surrounding environment of ultrasound imaging assembly 610 may be increased above the melting point of the materials used to construct protrusion 720 of tip member 710 and inner member 256, but be kept below the melting point of other components of ultrasound imaging assembly 610 including but not limited to support member 530, flexible substrate 214, and others. In some embodiments, the flexible substrate 214 may be composed of a thermal set material that will not melt during reflow, such as Kapton^{®} or any other suitable material. The range of settings during a reflow process may be configured based on visual inspection by a manufacturer of the ultrasound imaging assembly 610. In some embodiments, the range of settings during a reflow process may comprise the temperature of the environment during reflow, the areas or parts of ultrasound imaging assembly 610 to be included in the reflow process, or other features or characteristics. Other components may also be shielded to avoid melting during a reflow process. In some embodiments, shielding components may not be necessary due to characteristics and/or properties of the heat generating element used during a reflow process. For example, the travel distance of the heat may depend on the length of the heated die. During this reflow process, protrusion 720 and inner member 256 may begin a phase transition from a substantially solid material to a liquid material such that, once the temperature cools, the two components fuse together becoming one unitary structure and ensuring a very strong bond between tip member 710, inner member 256, and support member 530. In some embodiments in which protrusion 720 is constructed of a different material than tip member 710, protrusion 720 alone fuses with inner member 256 such that they become a unitary structure. The material of protrusion 720 may be selected with a melting point below the environmental temperature of the ultrasound imaging assembly 610 during the reflow process and the material of tip member 710 may be selected with a melting point above the environmental temperature. In this embodiment, only protrusion 720 may phase transition and fuse to inner member 256 while tip member 710 may retain its original shape.

At some point after tip member 710 is positioned in contact with support member 530 and ultrasound imaging assembly 610, and either before or after the reflow process previously described, adhesive 810 may be applied to the distal connection between tip member 710 and ultrasound imaging assembly 610. Adhesive 810 may fill gaps left between the outer surface 714 of tip member 710 and the inner surface of flexible substrate 214 in its rolled configuration. Adhesive 810 may also fill gaps left between the inner surface 724 of tip member 710 and surface 622 (Fig. 6) of support member 530 and may extend distally within tip member 710. Adhesive 810 may be any particular type of suitable adhesive, such as epoxy, cyanoacrylate, urethane adhesive, and/or acrylic adhesives, as well as others. Adhesive 810 may be liquid of any suitable viscosity. In some embodiments, adhesive 810 is not light cured (e.g., not UV cured).

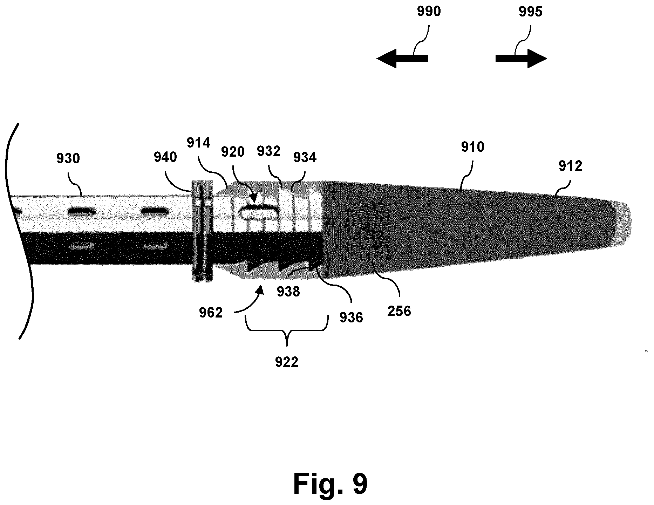

Fig. 9 is a partial cutaway view of another embodiment of the connection of the distal end of a support member 930 and the proximal end of a tip member 910, according to aspects of the present disclosure. Support member 930 may be similar to support member 530 presented previously and may serve similar functions as support member 530. Support member 930 may also be referred to as a unibody. Similar to support member 530, support member 930 may also be composed of a metallic material, such as stainless steel, or a non-metallic material. The support member 930 can be ferrule and may be manufactured according to any suitable process. Support member 930 may comprise one or more stands 940 extending radially from support member 930. These stands may be substantially similar to stands 640, 242 and 244 and may serve similar functions. Support member 930 may be of any suitable size.

In some embodiments, as shown in Fig. 9, support member 930 may comprise a recess 920 at the distal portion 962 of support member 930. Recess 920 may be substantially similar to recess 620 presented in Fig. 6. Like recess 620, recess 920 may be of any suitable shape. For example, as shown in Fig. 9, recess 920 may be of a generally oval shape. In other embodiments, recess 920 may be circular, or have a cross-sectional profile of any other suitable shape. Recess 920 may extend completely through the surface of support member 930 such that the outer environment of support member 930 may be in direct contact with the inner lumen within the axial center of support member 930. Recess 920 may receive a protrusion positioned on the inner surface of tip member 910. Such a protrusion may be substantially similar to protrusion 720. Recess 920 may allow a protrusion on the inner surface of tip member 910 to come into direct contact with the outer surface of inner member 256.

In some embodiments, as shown in Fig. 9, support member 930 may further comprise one or more ridges 932 positioned around the outer surface of the distal portion 962 of support member 930. Ridges 932 may also be referred to as barbs, spikes, anchors, flanges, collars, or ribs. In some embodiments, ridges 932 and support member 930 may be integrally formed as a unitary structure. Ridges 932 may be of any suitable shape. As shown in Fig. 9, in some embodiments, ridges 932 may comprise two outer surfaces, distal surface 936 and proximal surface 938. Distal surface 936 may be sloped such that the outer diameter of support member 930 is substantially similar to the overall outer diameter of support member 930 at a distal point of a ridge 932 of support member 930 and then gradually increases to a larger diameter at a proximal point on ridge 932. Surface 938 may extend in a substantially orthogonal direction to the outer surface of support member 930 as shown in Fig. 9. This configuration of a sloped distal surface 936 and an oblique proximal surface 938 results in a more secure connection between tip member 710 and support member 930 by allowing tip member to move in a proximal direction, as indicated by arrow 990, when being connected to support member 930 but not allowing tip member to move in a distal direction, as indicated by arrow 995. In other embodiments, surface 938 may be a sloped surface as well. In still other embodiments, ridges 932 may comprise additional surfaces and may be of any suitable cross-sectional shape. For example, the cross-sectional profile of ridges 932 may be substantially rectangular or semi-circular among other geometric and non-geometric shapes. In some embodiments, ridges 932 may extend completely around the outer surface of support member 930 at a longitudinal point along support member 930. In other embodiments, ridges 932 may only extend partially around outer surface of support member 930. For example, ridges 932 may extend around substantially half of the outer surface of support member 930, substantially a quarter of the outer surface of support member 930 or around any other suitable portion of support member 930. In addition, at the same longitudinal point along support member 930, there may be positioned more than one such ridge 932 extending along some fraction of the outer circumference of support member 930.

As shown in Fig. 9, the support member 930 can include multiple ridges 932. Three such ridges 932 are depicted in Fig. 9, however, fewer or more ridges 932 may be used in the design of support member 930. For example, one, two, three, four, five, or more ridges 932 may be placed along the outer surface of the distal portion 962 of support member 930. Ridges 932 may be in any position relative to recess 920. As shown in Fig. 9, recess 920 may overlap with one or more of ridges 932, however, in other embodiments, recess 920 may be positioned between, proximal or distal of one or more ridges 932.

As also shown in Fig. 9, tip member 910 may comprise one or more grooves 934. Grooves 934 may also be referred to as troughs, channels, indentations, notches, or other suitable terms. In some embodiments, grooves 934 may be of a substantially similar shape as ridges 932 such that ridges 932 may be received into grooves 934 when the proximal end of tip member 910 is brought into contact with the distal end of support member 930. As with ridges 932, grooves 934 be of any suitable cross-sectional shape. For example, the cross-sectional profile of grooves 934 may be substantially rectangular or semi-circular among other geometric and non-geometric shapes, Similarly, grooves 934 may extend completely around the inner surface of tip member 910 at a longitudinal point along tip member 910. In other embodiments, grooves 934 may only extend partially around inner surface of the tip member 910. In addition, at the same longitudinal point along the inner surface of tip member 910, there may be positioned more than one such groove 934 extending along some fraction of the inner circumference of tip member 910. In addition, although three such grooves 934 are depicted in Fig. 9, fewer or more grooves 934 may be used in the design of tip member 910. For example, one, two, three, four, five, or more grooves 934 may be placed along the inner surface of the proximal portion of tip member 910. Further, although not depicted in the partial cutaway view of tip member 910 in Fig. 9, tip member 910 may comprise one or more protrusions substantially similar to protrusion 720 previously discussed. These protrusions may be positioned at any suitable location within the inner surface of tip member 910 and in any position relative to grooves 934.

In another embodiment of the disclosed invention, the location of ridges 932 and grooves 934 may be reversed. For example, ridges may be located on the inner surface of the proximal portion of tip member 910. These ridges may extend inwardly radially from the inner surface of tip member 910 so as to protrude into the inner lumen created by tip member 910. In contrast, the distal portion 962 of support member 930 may comprise corresponding grooves similar to grooves 934 depicted in Fig. 9. However, these grooves may be positioned on the outer surface of the distal portion 962 of support member 930 so as to create a recess or void extending inwardly radially into the outer surface of support member 930.

In still other embodiments, the outer surface of support member 930 may comprise one or more of both ridges and grooves as previously described and the inner surface of tip member 910 may similarly comprise one or more of both ridges and grooves as previously described. This plurality of both ridges and grooves positioned on both support member 930 and tip member 910 may be configured to receive one another or interlock with one another creating a substantially stronger coupling of support member 930 and tip member 910.

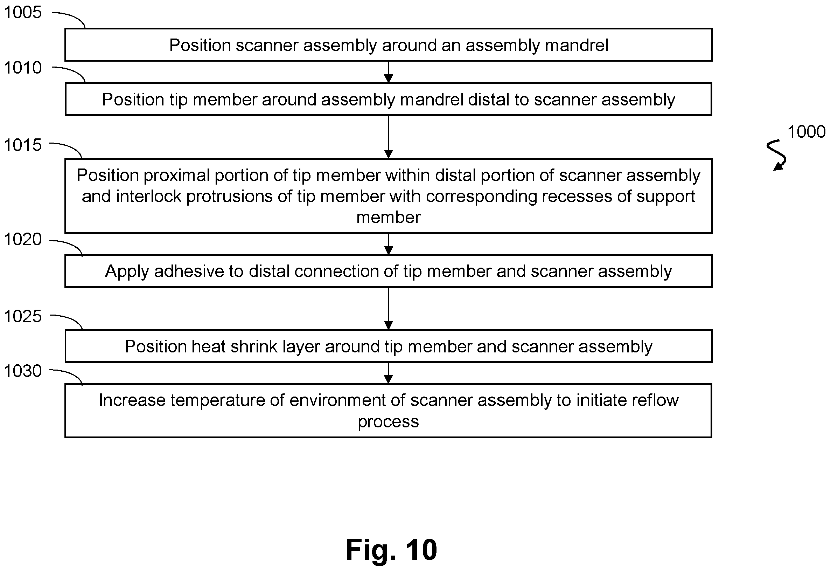

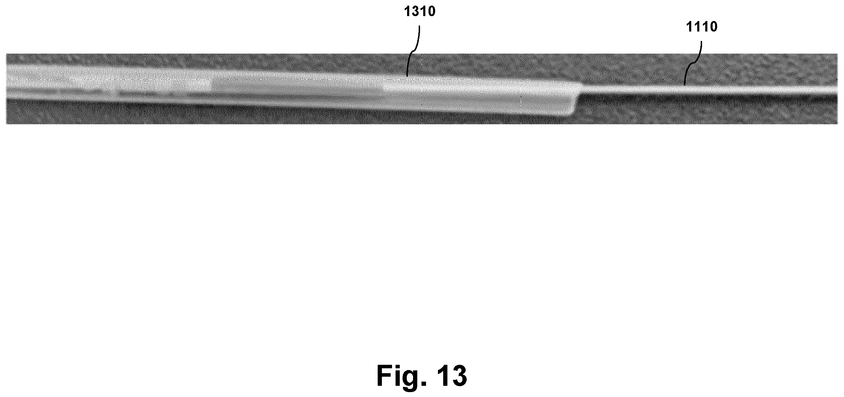

Fig. 10 is a flow diagram of a method 1000 of a phase of assembling an intraluminal imaging device 102 according to an embodiment of the present disclosure. The method 1000 can include mechanically coupling tip member 710 to intraluminal imaging device 102. As illustrated, method 1000 includes a number of enumerated steps, but embodiments of method 1000 may include additional steps before, after, or in between the enumerated steps. In some embodiments, one or more of the enumerated steps may be omitted, performed in a different order, or performed concurrently. The steps of method 1000 can be carried out by a manufacturer of the intraluminal imaging device 102 and/or a manufacturer of any other component discussed in the present disclosure. Method 1000 will be described with reference to Figs. 13-16, which are side views of various components of the ultrasound imaging assembly 102 during various steps of manufacturing. For example, Figs. 13-16 illustrate assembly steps for various components of the ultrasound imaging assembly 102, such as the connection between the intraluminal imaging device 102 and the tip member 710.

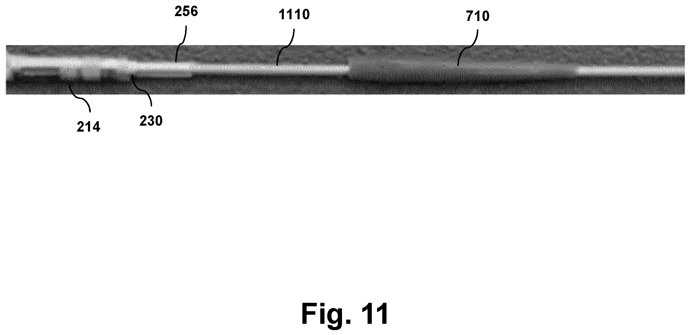

At step 1005, method 1000 includes positioning scanner assembly or ultrasound imaging assembly 110 around an assembly mandrel 1110 (Fig. 11). An assembly mandrel 1110 may be used to support the ultrasound imaging assembly 110 during various stages of manufacturing. Assembly mandrel 1110 may be of any suitable length. The diameter of assembly mandrel 1110 may correspond to the inner diameter of the inner member 256 or may differ. In other embodiments, ultrasound imaging assembly 110 may be constructed without the use of assembly mandrel 1110. Assembly mandrel 1110 may also be referred to as a support shaft or support rod. Assembly mandrel 1110 may be constructed of any suitable material, including metallic and non-metallic materials. In addition, assembly mandrel 1110 may be a rigid structure or semi-rigid structure.

At step 1010, method 1000 includes positioning tip member 710 around assembly mandrel 1110 at some point distal to scanner assembly or ultrasound imaging assembly 110 as shown in Fig. 11. Fig. 11 is a side view of the intraluminal imaging device 102 and tip member 710 positioned around an assembly mandrel 1110 before coupling the tip member 710 to the scanner assembly 110, according to aspects of the present disclosure.

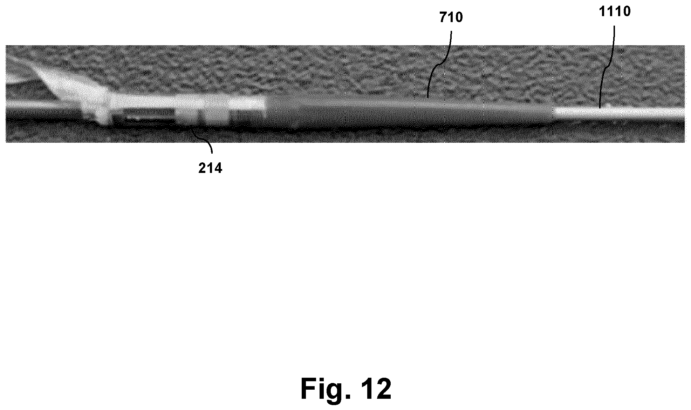

At step 1015, method 1000 includes positioning the proximal portion of tip member 710 within the distal portion of ultrasound imaging assembly or scanner assembly 110 and interlocking protrusions 720 of tip member 710 with corresponding recesses 620 of support member 530 as shown in Fig. 12. Fig. 12 is a side view of the ultrasound imaging assembly or scanner assembly 110 and a tip member 710 positioned around an assembly mandrel 1110 after coupling the tip member 710 to the scanner assembly 110, according to aspects of the present disclosure. As previously discussed, when tip member 710 is placed in connection or direct contact with scanner assembly 110, protrusions 720 of tip member 710 may be received into corresponding recesses 620 of support member 530 creating a strong mechanical connection between support member 530 and tip member 710. Protrusions 720 may also be in direct contact with inner member 256 such that during a subsequent reflow process, protrusions 720 and inner member 256 fuse together and becoming a unitary structure.

At step 1020, method 1000 includes applying adhesive 810 to the distal connection of the tip member 710 and ultrasound imaging assembly or scanner assembly 110. Adhesive 810 may additionally be applied to the connection of tip member 710 and scanner assembly 110 and fill gaps between the outer surface 714 of tip member 710 and the inner surface of flexible substrate 214 in its rolled configuration and gaps between the inner surface 724 of tip member 710 and outer surface 622 of support member 530. Adhesive 810 applied in such a manner may increase the strength of the connection between tip member 710 and ultrasound imaging assembly 110 as described in relation to Fig. 8.

At step 1025, method 1000 includes positioning a heat shrink layer 1310 around tip member 710 and ultrasound imaging assembly or scanner assembly 110. Fig. 13 is a side view of a heat shrink layer 1310 positioned around an ultrasound imaging assembly or scanner assembly 110 and a tip member 710 supported by an assembly mandrel 1110, according to aspects of the present disclosure. A heat shrink layer 1310 may be applied around the scanner assembly 110 and related components prior to a reflow process. Heat shrink layer 1310 may be constructed of any one of a variety of thermoplastics, including polyolefin, polyvinyl chloride, polychloroprene (e.g., NEOPRENE^{®}), among others. Heat shrink layer 1310 may be positioned at a location around the outer surface of scanner assembly 110 and then subjected to increased temperature, causing heat shrink layer 1310 to contract inwardly around scanner assembly 110. Heat shrink layer 1310 may be used to shield components such as the inner member 256, outer member 254, scanner assembly 110, or other components that are incompatible with high temperature exposure during a reflow process. In some embodiments, heat shrink layer 1310 may also serve other purposes as well. After a reflow process is completed, heat shrink layer 1310 may be removed from the intraluminal imaging device.

At step 1030, method 1000 includes increasing the temperature of the environment of the ultrasound imaging assembly or scanner assembly 110 to initiate a reflow process. As previously described, during a reflow process, the temperature of the surrounding environment of ultrasound imaging assembly or scanner assembly 110 may be increased above the melting point of the materials used to construct protrusion 720 of tip member 710 and inner member 256, but be kept below the melting point of other components of scanner assembly 110. Protrusion 720 and inner member 256 may then begin a phase transition such that the two components fuse together becoming one unitary structure and ensuring a very strong bond between tip member 710, inner member 256 and support member 530. In some embodiments, heat shrink layer 1310 may be removed at this phase of assembly. In other embodiments, heat shrink layer 1310 may not be removed. In some embodiments, additional steps may be performed to reach a final product including applying hydrophobic or hydrophilic coatings to various components or portions of the scanner assembly 110 and its associated components. Other steps may be taken in different embodiments as well.

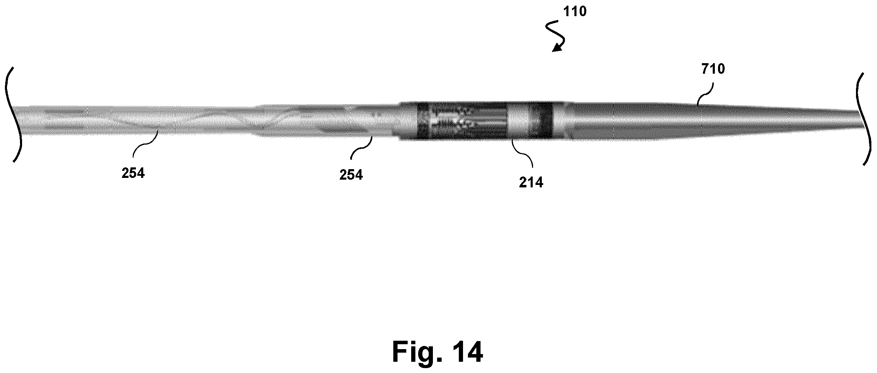

Fig. 14 is a side view of an ultrasound imaging assembly or scanner assembly 110 after assembly, according to aspects of the present disclosure. Ultrasound imaging assembly or scanner assembly 110 is connected at a distal end to tip member 710 and connected at a proximal end to outer member 254. Flexible substrate 214 is depicted in Fig. 14 wrapped around a support member. A plurality of conductors, transmission line bundle, or cables 112 are also depicted connected at a proximal end of scanner assembly 110 extending proximally from the assembly.

Embodiments of the presently disclosed invention may comprise protrusions and/or corresponding recesses/grooves at any suitable location of the intraluminal device. For example, one or more protrusion(s) and/or one or more recesses/grooves can be provided on tip member 710, the scanner assembly 110, the flexible substrate 214, the support member 230, the inner member 256, and/or any other component described in the present application. The protrusions and/or recesses/grooves can have any suitable size and shape, orientation, and/or direction of extension (radially inward, radially outward, proximally, distally, obliquely, perpendicularly, etc.) For example, a flexible substrate 214 can include a protrusion that extends radially inward and is received within a recess/groove of the support member 230, the tip member 710, and/or the inner member 256. For example, the inner member 256 can include a protrusion that extends radially outward and is received within a recess/groove of the flexible substrate 214, the support member 230, and/or the tip member 710.

One or more embodiments of the present disclosure advantageously increase coupling strength between components. This advantageously ensures that the components cannot come apart. This improves patient safety by minimizing and/or eliminating the possibility of components separating while the intraluminal device is within the patient's body. Such strength can be added as the result of mechanical engagement between components (e.g., physical contact resulting from the protrusion of one component being received within a recess/groove of another component). In some embodiments, the material of multiple components can be joined together. For example, polymer material(s) can be fused or melted together during a reflow process. In some embodiments, the mechanical engagement between components is supported by adhesive surrounding and in contact with the joint.

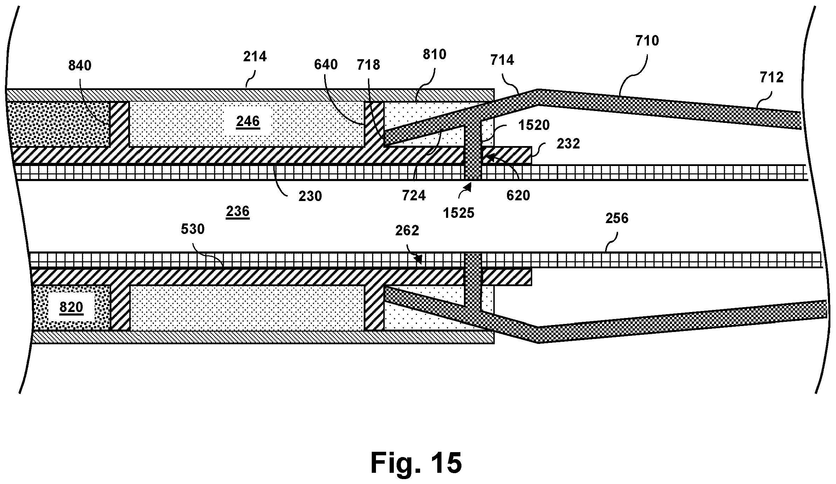

Fig. 15 is a diagrammatic cross-sectional view of an embodiment of the connection between components at the distal portion of the intraluminal imaging device, according to aspects of the present disclosure. Fig. 15 includes a protrusion 1520 of the tip member 710. Protrusion 1520 may be substantially similar to protrusion 720 previously mentioned or may differ. As shown in Fig. 15, protrusion 1520 may be configured to be received into recess 620 of support member 230. Protrusion 1520 may also be configured to be received into an additional recess 1525 within inner member 256, such that the tip of protrusion 1520 may be in direct contact with lumen 236 created by inner member 256. In other embodiments, recess 1525 may not extend completely through inner member 256 as shown in Fig. 15. For example, recess 1525 may only extend part way through inner member 256 such as to extend into the outer surface of inner member 256 but not through the inner surface of inner member 256. In either of these embodiments, however, during a reflow process, protrusion 1520 fuses with inner member 256 so as to create a strong bond between the two components and forming one unitary structure.

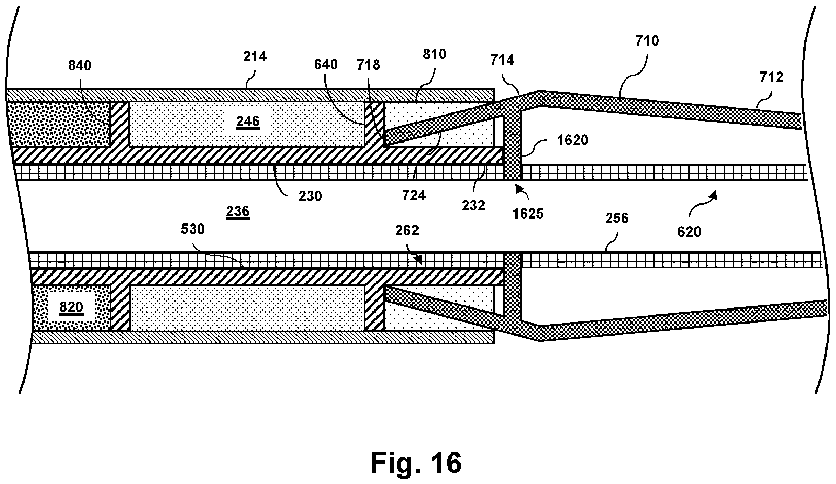

Fig. 16 is a diagrammatic cross-sectional view of an embodiment of the connection between components at the distal portion of the intraluminal imaging device, according to aspects of the present disclosure. Fig. 16 includes a protrusion 1620 of the tip member 710. Protrusion 1620 may be substantially similar to protrusion 720 and/or protrusion 1520 previously mentioned or may differ. As shown in Fig. 16, protrusion 1620 may be configured to be received into recess 1625 of inner member 256. Recess 1625 may be substantially similar to recess 1525 previously mentioned. For example, recess 1625 may extend completely through inner member 256 such that recess 1625 may be detected on both the outer surface and inner surface of inner member 256. In addition, in other embodiments, recess 1625 may only extend part way through inner member 256 such as to extend into the outer surface of inner member 256 but not through the inner surface of inner member 256. In either of these embodiments, however, during a reflow process, protrusion 1620 fuses with inner member 256 so as to create a strong bond between the two components and forming one unitary structure. As shown in Fig. 16, protrusion 1620 may be positioned at some location distal of support member 230 so as to not extend through any recess on or within support member 230. In some embodiments, the distal tip of support member 230 may abut protrusion 1620. In other embodiments, protrusion 1620 may be spaced from the support member 230 so as not to be in contact with support member 230.

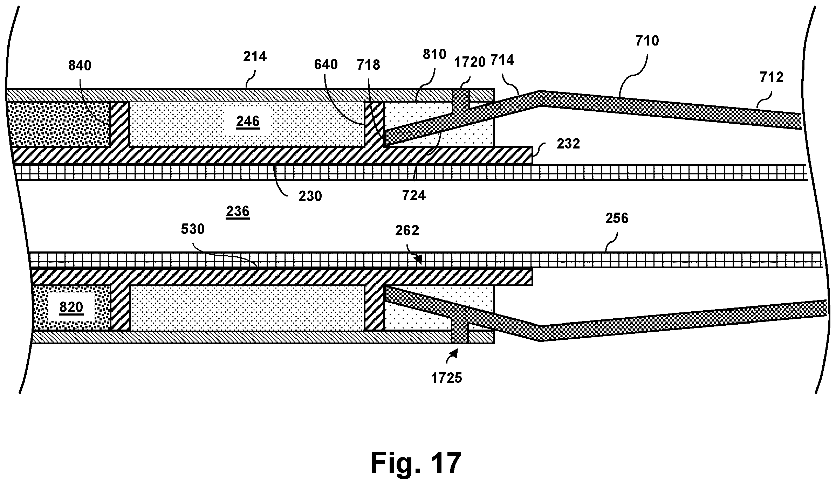

Fig. 17 is a diagrammatic cross-sectional view of an embodiment of the connection between components at the distal portion of the intraluminal imaging device, according to aspects of the present disclosure. Fig. 17 includes a protrusion 1720 of the tip member 710. Protrusion 1720 may be substantially similar to protrusion 720, protrusion 1520, and/or protrusion 1620 except that protrusion 1720 may extend radially outward from the outward surface of tip member 710. As shown in Fig. 17, protrusion 1720 may be configured to be received into recess 1725 of flexible substrate 214. Recess 1725 may be substantially similar to recess 1525 and/or recess 1625 previously mentioned but may be positioned within support member 214. For example, recess 1725 may extend completely through flexible substrate 214 such that recess 1725 may be detected on both the outer surface and inner surface of flexible substrate 214. In addition, in other embodiments, recess 1725 may only extend part way through flexible substrate 214 so as to extend into the inner surface of flexible substrate 214 but not through the outer surface of flexible substrate 214. In some embodiments, during a reflow process, protrusion 1720 fuses with flexible substrate 214 so as to create a strong bond between the two components and forming one unitary structure. In other embodiments, protrusion 1720 may not bond with flexible substrate 214 during a reflow process. Protrusion 1720 may be positioned at any suitable location and a corresponding recess within flexible substrate 214 may be positioned at any suitable location within, on, or around scanner assembly 110.

In other embodiments, an additional protrusion may extend from tip member 710 in a proximal direction towards stand 640 of support member 230. Such a protrusion may be received into a corresponding recess positioned within stand 640 of support member 230. Such a recess may be substantially similar to recess 235 of Fig. 4 or may differ. Such a configuration may provide a stronger connection between tip member 710 and the scanner assembly 110. In some embodiments, a protrusion may extend distally from the stand 640 and be received within a recess of the tip member 710.

While some embodiments are described in the context of an intraluminal ultrasound imaging device, it is understood that the present disclosure contemplates any type of intraluminal device (e.g., catheter, guide wire, guide catheter, etc.). For example, the intraluminal device can include a pressure sensor, flow sensor, temperature sensor, and/or electrode(s). The intraluminal device can include any suitable type of optical sensor. Any type of intraluminal imaging is contemplated, including intraluminal devices with an optical coherence tomography (OCT) imaging element and/or an intracardiac echocardiography (ICE) transducer array. One or more sensing devices of the intraluminal device can obtain physiological data (pressure, flow, temperature, images, etc.) associated with the body lumen in which intraluminal device is positioned. In some embodiments, the intraluminal device is a diagnostic device. In some embodiments, the intraluminal device is a therapeutic device that delivers a therapy to the body lumen. Such a therapeutic intraluminal device can have a treatment component at a distal portion (e.g., a balloon, an ablation electrode, a laser, an atherectomy blade, etc.)

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure.

## Claims

1. An intraluminal imaging device (102), comprising:
a flexible elongate member (121) configured to be positioned within a body lumen of a patient, the flexible elongate member comprising a proximal portion and a distal portion;
an ultrasound imaging assembly disposed at the distal portion of the flexible elongate member and configured to obtain ultrasound data while positioned within the body lumen; and
a tip member (710) coupled to the ultrasound imaging assembly, wherein the tip member comprises a polymer protrusion (720),
wherein the ultrasound imaging assembly comprises a recess (620, 920, 1525, 1625, 1725) exposing a polymer portion of the ultrasound imaging assembly, and wherein the protrusion received within the recess is fused to the exposed polymer portion of the ultrasound imaging assembly to form a unitary structure.

2. The intraluminal imaging device of claim 1, wherein the ultrasound imaging assembly comprises a support member (230) and a flexible substrate (214) positioned around the support member, wherein the support member comprises the recess (620).

3. The intraluminal imaging device of claim 2,
wherein the support member comprises a proximal portion and a distal portion, wherein the recess is disposed at the distal portion of the support member,
wherein the tip member comprises a proximal portion and a distal portion, wherein the protrusion is disposed at the proximal portion of the tip member,
wherein the distal portion of the support member is coupled to the proximal portion of the tip member.

4. The intraluminal imaging device of claim 2, wherein the flexible elongate member comprises an inner member (256) extending through the support member such that the recess exposes a portion of the inner member.

5. The intraluminal imaging device of claim 4, wherein the support member comprises a first surface and an opposite, second surface, wherein the recess extends from the first surface to the second surface.

6. The intraluminal imaging device of claim 4, wherein the tip member comprises a first polymer and the inner member comprises a second polymer.

7. The intraluminal imaging device of claim 2, wherein the protrusion extends radially inward.

8. The intraluminal imaging device of claim 2, wherein the protrusion extends radially outward.

9. The intraluminal imaging device of claim 2, wherein the support member comprises a plurality of recesses, and wherein the tip member comprises a plurality of protrusions, wherein the plurality of protrusions is received within the plurality of recesses.

10. The intraluminal imaging device of claim 2, wherein the tip member comprises an outer surface, wherein the outer surface comprises a first sloped portion and an opposite, second sloped portion, and wherein the protrusion is aligned with the first sloped portion.

11. The intraluminal imaging device of claim 1, comprising:
wherein the ultrasound imaging assembly comprises:
a support member comprising a first stand, a second stand, a third stand, and a ridge;
a flexible substrate positioned around the first stand, the second stand, and the third stand, wherein the flexible substrate comprises a plurality of integrated circuit chips and an ultrasound transducer assembly;
a filling material between the first stand, the second stand, and a portion of the flexible substrate comprising the plurality of integrated circuit chips; and
an acoustic backing material between the second stand, the third stand, and a portion of the flexible substrate comprising the ultrasound transducer assembly; and
wherein the tip member comprises a groove (934) for receiving the ridge (932).

12. The intraluminal imaging device of claim 11, wherein the groove of the tip member is configured to engage the ridge of the support member to allow relative movement between the tip member and support member in a first direction and restrict the relative movement in an opposite, second direction.

13. The intraluminal imaging device of claim 12,
wherein the groove of the tip member comprises a proximal portion and a distal portion, wherein the proximal portion of the groove has a first diameter larger than a second diameter of the distal portion of the groove, and
wherein the ridge of the support member comprises a proximal portion and a distal portion, wherein the proximal portion of the ridge has a third diameter larger than a fourth diameter of the distal portion of the ridge.

14. The intraluminal imaging device of claim 11, wherein the support member comprises a plurality of ridges, wherein the tip member comprises a plurality of grooves receiving the plurality of ridges.

15. The intraluminal imaging device of claim 4, being a catheter,
wherein the flexible elongate member (121) further comprises an outer polymer member positioned around the inner member of polymer;
wherein the flexible substrate comprises an ultrasound transducer array, wherein the support member comprises a plurality of recesses exposing portions of the inner member extending through support member;
wherein the tip member is of polymer and comprises a plurality of protrusions received within the plurality of recesses, and wherein the plurality of protrusions is fused to the exposed portions of the polymer inner member.

## Patentansprüche

1. Intraluminale Bildgebungsvorrichtung (102), umfassend:
ein flexibles, längliches Element (121), das konfiguriert ist, in einem Körperlumen eines Patienten positioniert zu werden, wobei das flexible, längliche Element einen proximalen Abschnitt und einen distalen Abschnitt umfasst;
eine Ultraschallbildgebungsanordnung, die am distalen Abschnitt des flexiblen länglichen Elements angeordnet ist und konfiguriert ist, um Ultraschalldaten zu erhalten, während sie im Körperlumen positioniert ist; und
ein Spitzenelement (710), das mit der Ultraschallbildgebungsanordnung gekoppelt ist, wobei das Spitzenelement einen Polymervorsprung (720) umfasst,
wobei die Ultraschallbildgebungsanordnung eine Aussparung (620, 920, 1525, 1625, 1725) umfasst, die einen Polymerabschnitt der Ultraschallbildgebungsanordnung freilegt, und wobei der in der Aussparung aufgenommene Vorsprung mit dem freigelegten Polymerabschnitt der Ultraschallbildgebungsanordnung verschmolzen ist, um eine einheitliche Struktur zu bilden.

2. Intraluminale Bildgebungsvorrichtung nach Anspruch 1, wobei die Ultraschallbildgebungsanordnung ein Trägerelement (230) und ein flexibles Substrat (214) umfasst, das um das Trägerelement herum positioniert ist, wobei das Trägerelement die Aussparung (620) umfasst.

3. Intraluminale Bildgebungsvorrichtung nach Anspruch 2,
wobei das Trägerelement einen proximalen Abschnitt und einen distalen Abschnitt umfasst, wobei die Aussparung am distalen Abschnitt des Trägerelements angeordnet ist,
wobei das Spitzenelement einen proximalen Abschnitt und einen distalen Abschnitt umfasst, wobei der Vorsprung am proximalen Abschnitt des Spitzenelements angeordnet ist,
wobei der distale Abschnitt des Trägerelements mit dem proximalen Abschnitt des Spitzenelements gekoppelt ist.

4. Intraluminale Bildgebungsvorrichtung nach Anspruch 2, wobei das flexible, längliche Element ein inneres Element (256) umfasst, das sich durch das Trägerelement hindurch erstreckt, sodass die Aussparung einen Abschnitt des inneren Elements freilegt.

5. Intraluminale Bildgebungsvorrichtung nach Anspruch 4, wobei das Trägerelement eine erste Oberfläche und eine gegenüberliegende zweite Oberfläche umfasst, wobei sich die Aussparung von der ersten Oberfläche zur zweiten Oberfläche erstreckt.

6. Intraluminale Bildgebungsvorrichtung nach Anspruch 4, wobei das Spitzenelement ein erstes Polymer umfasst und das innere Element ein zweites Polymer umfasst.

7. Intraluminale Bildgebungsvorrichtung nach Anspruch 2, wobei sich der Vorsprung radial nach innen erstreckt.

8. Intraluminale Bildgebungsvorrichtung nach Anspruch 2, wobei sich der Vorsprung radial nach außen erstreckt.

9. Intraluminale Bildgebungsvorrichtung nach Anspruch 2, wobei das Trägerelement eine Vielzahl von Aussparungen umfasst und wobei das Spitzenelement eine Vielzahl von Vorsprüngen umfasst, wobei die Vielzahl von Vorsprüngen innerhalb der Vielzahl von Aussparungen aufgenommen ist.

10. Intraluminale Bildgebungsvorrichtung nach Anspruch 2, wobei das Spitzenelement eine Außenoberfläche umfasst, wobei die Außenoberfläche einen ersten geneigten Abschnitt und einen gegenüberliegenden zweiten geneigten Abschnitt umfasst und wobei der Vorsprung mit dem ersten geneigten Abschnitt ausgerichtet ist.

11. Intraluminale Bildgebungsvorrichtung nach Anspruch 1, umfassend:
wobei die Ultraschallbildgebungsanordnung umfasst:
ein Trägerelement, das einen ersten Ständer, einen zweiten Ständer, einen dritten Ständer und eine Rippe umfasst;
ein flexibles Substrat, das um den ersten Ständer, den zweiten Ständer und den dritten Ständer herum positioniert ist, wobei das flexible Substrat eine Vielzahl von integrierten Schaltkreischips und eine Ultraschallwandleranordnung umfasst;
ein Füllmaterial zwischen dem ersten Ständer, dem zweiten Ständer und einem Abschnitt des flexiblen Substrats, das die Vielzahl von integrierten Schaltkreischips umfasst; und
ein akustisches Trägermaterial zwischen dem zweiten Ständer, dem dritten Ständer und einem Abschnitt des flexiblen Substrats, das die Ultraschallwandleranordnung umfasst; und wobei das Spitzenelement eine Nut (934) zur Aufnahme der Rippe (932) umfasst.

12. Intraluminale Bildgebungsvorrichtung nach Anspruch 11, wobei die Nut des Spitzenelements konfiguriert ist, um mit der Rippe des Trägerelements in Eingriff zu kommen, um eine relative Bewegung zwischen dem Spitzenelement und Trägerelement in einer ersten Richtung zu ermöglichen und die relative Bewegung in einer entgegengesetzten, zweiten Richtung einzuschränken.

13. Intraluminale Bildgebungsvorrichtung nach Anspruch 12,
wobei die Nut des Spitzenelements einen proximalen Abschnitt und einen distalen Abschnitt umfasst, wobei der proximale Abschnitt der Nut einen ersten Durchmesser aufweist, der größer ist als ein zweiter Durchmesser des distalen Abschnitts der Nut, und
wobei die Rippe des Trägerelements einen proximalen Abschnitt und einen distalen Abschnitt umfasst, wobei der proximale Abschnitt der Rippe einen dritten Durchmesser aufweist, der größer ist als ein vierter Durchmesser des distalen Abschnitts der Rippe.

14. Intraluminale Bildgebungsvorrichtung nach Anspruch 11, wobei das Trägerelement eine Vielzahl von Rippen umfasst, wobei das Spitzenelement eine Vielzahl von Nuten umfasst, die die Vielzahl von Rippen aufnehmen.

15. Intraluminale Bildgebungsvorrichtung nach Anspruch 4, die ein Katheter ist,
wobei das flexible längliche Element (121) weiter ein äußeres Polymerelement umfasst, das um das innere Polymerelement herum positioniert ist;
wobei das flexible Substrat ein Ultraschallwandlerarray umfasst, wobei das Trägerelement eine Vielzahl von Aussparungen umfasst, die Abschnitte des inneren Elements freilegen, die sich durch das Trägerelement hindurch erstrecken;
wobei das Spitzenelement aus Polymer ist und eine Vielzahl von Vorsprüngen umfasst, die in der Vielzahl von Aussparungen aufgenommen sind, und wobei die Vielzahl von Vorsprüngen mit den freigelegten Abschnitten des inneren Polymerelements verschmolzen ist.

## Revendications

1. Dispositif d'imagerie intraluminale (102), comprenant :
un élément allongé flexible (121) configuré pour être positionné à l'intérieur d'une lumière corporelle d'un patient, l'élément allongé flexible comprenant une partie proximale et une partie distale ;
un ensemble d'imagerie par ultrasons disposé au niveau de la partie distale de l'élément allongé flexible et configuré pour obtenir des données ultrasonores pendant qu'il est positionné à l'intérieur de la lumière corporelle ; et
un élément de pointe (710) couplé à l'ensemble d'imagerie par ultrasons, dans lequel l'élément de pointe comprend une saillie en polymère (720),
dans lequel l'ensemble d'imagerie par ultrasons comprend un évidement (620, 920, 1525, 1625, 1725) exposant une partie polymère de l'ensemble d'imagerie par ultrasons, et dans lequel la saillie reçue dans l'évidement est fusionnée à la partie polymère exposée de l'ensemble d'imagerie par ultrasons pour former une structure unitaire.

2. Dispositif d'imagerie intraluminale selon la revendication 1, dans lequel l'ensemble d'imagerie par ultrasons comprend un élément de soutien (230) et un substrat flexible (214) positionné autour de l'élément de soutien, dans lequel l'élément de soutien comprend l'évidement (620).

3. Dispositif d'imagerie intraluminale selon la revendication 2,
dans lequel l'élément de soutien comprend une partie proximale et une partie distale, dans lequel l'évidement est disposé au niveau de la partie distale de l'élément de soutien,
dans lequel l'élément de pointe comprend une partie proximale et une partie distale, dans lequel la saillie est disposée au niveau de la partie proximale de l'élément de pointe,
dans lequel la partie distale de l'élément de soutien est couplée à la partie proximale de l'élément de pointe.

4. Dispositif d'imagerie intraluminale selon la revendication 2, dans lequel l'élément allongé flexible comprend un élément interne (256) s'étendant à travers l'élément de soutien de telle sorte que l'évidement expose une partie de l'élément interne.

5. Dispositif d'imagerie intraluminale selon la revendication 4, dans lequel l'élément de soutien comprend une première surface et une seconde surface opposée, dans lequel l'évidement s'étend de la première surface à la seconde surface.

6. Dispositif d'imagerie intraluminale selon la revendication 4, dans lequel l'élément de pointe comprend un premier polymère et l'élément interne comprend un second polymère.

7. Dispositif d'imagerie intraluminale selon la revendication 2, dans lequel la saillie s'étend radialement vers l'intérieur.

8. Dispositif d'imagerie intraluminale selon la revendication 2, dans lequel la saillie s'étend radialement vers l'extérieur.

9. Dispositif d'imagerie intraluminale selon la revendication 2, dans lequel l'élément de soutien comprend une pluralité d'évidements, et dans lequel l'élément de pointe comprend une pluralité de saillies, dans lequel la pluralité de saillies est reçue à l'intérieur de la pluralité d'évidements.

10. Dispositif d'imagerie intraluminale selon la revendication 2, dans lequel l'élément de pointe comprend une surface externe, dans lequel la surface externe comprend une première partie inclinée et une seconde partie inclinée opposée, et dans lequel la saillie est alignée avec la première partie inclinée.

11. Dispositif d'imagerie intraluminale selon la revendication 1, comprenant :
dans lequel l'ensemble d'imagerie par ultrasons comprend :
un élément de soutien comprenant un premier support, un deuxième support, un troisième support et une arête ;
un substrat flexible positionné autour du premier support, du deuxième support et du troisième support, dans lequel le substrat flexible comprend une pluralité de puces de circuit intégré et un ensemble transducteur à ultrasons ;
un matériau de remplissage entre le premier support, le deuxième support et une partie du substrat flexible comprenant la pluralité de puces de circuit intégré ; et
un matériau de support acoustique entre le deuxième support, le troisième support et une partie du substrat flexible comprenant l'ensemble transducteur à ultrasons ; et dans lequel l'élément de pointe comprend une rainure (934) destinée à recevoir l'arête (932).

12. Dispositif d'imagerie intraluminale selon la revendication 11, dans lequel la rainure de l'élément de pointe est configurée pour venir en prise avec l'arête de l'élément de soutien pour permettre un déplacement relatif entre l'élément de pointe et l'élément de soutien dans une première direction et restreindre le déplacement relatif dans une seconde direction opposée.

13. Dispositif d'imagerie intraluminale selon la revendication 12,
dans lequel la rainure de l'élément de pointe comprend une partie proximale et une partie distale, dans lequel la partie proximale de la rainure présente un premier diamètre supérieur à un deuxième diamètre de la partie distale de la rainure, et
dans lequel l'arête de l'élément de soutien comprend une partie proximale et une partie distale, dans lequel la partie proximale de l'arête présente un troisième diamètre supérieur à un quatrième diamètre de la partie distale de l'arête.

14. Dispositif d'imagerie intraluminale selon la revendication 11, dans lequel l'élément de soutien comprend une pluralité d'arêtes, dans lequel l'élément de pointe comprend une pluralité de rainures recevant la pluralité d'arêtes.

15. Dispositif d'imagerie intraluminale selon la revendication 4, étant un cathéter,
dans lequel l'élément allongé flexible (121) comprend en outre un élément polymère externe positionné autour de l'élément polymère interne ;
dans lequel le substrat flexible comprend un réseau de transducteurs à ultrasons, dans lequel l'élément de soutien comprend une pluralité d'évidements exposant des parties de l'élément interne s'étendant à travers l'élément de soutien ;
dans lequel l'élément de pointe est en polymère et comprend une pluralité de saillies reçues à l'intérieur de la pluralité d'évidements, et dans lequel la pluralité de saillies est fusionnée aux parties exposées de l'élément interne en polymère.
